# EUROPEAN PATENT APPLICATION

(11) **EP 4 410 843 A1**
(43) Date of publication of application: **07.08.2024**
(21) Application number: 22875230.9
(22) Date of filing: 30.11.2022
(51) Int. Cl.: C07K 19/00, C07K 16/10, C12N 15/70, G01N 33/569

(54) **RECOMBINANT FUSION PROTEIN DERIVED FROM HR REGION OF S2 PROTEIN OF SARS-COV-2 AND APPLICATION OF RECOMBINANT FUSION PROTEIN**

(30) Priority: 01.10.2021 CN 202111167024
(71) Applicant: Eternivax Biomedical, Inc, Shanghai 200131 (CN)
(72) Inventor: PANG, Wei, Kunming, Yunnan 650223 (CN); ZHENG, Yongtang, Kunming, Yunnan 650223 (CN); HE, Wenqiang, Kunming, Yunnan 650223 (CN); HE, Xiaoyan, Kunming, Yunnan 650223 (CN); LUO, Ronghua, Kunming, Yunnan 650223 (CN); LU, Ying, Kunming, Yunnan 650223 (CN); SHEN, Fan, Kunming, Yunnan 650223 (CN)
(74) Representative: Lavoix
(86) International application number: PCT/CN2022/135269
(87) International publication number: WO 2023/051850

(57) **Abstract**

Disclosed are a recombinant fusion protein derived from an HR region of an S2 protein of SARS-CoV-2 and an application of the recombinant fusion protein. The SARS-CoV-2 recombinant fusion protein is a recombinant fusion protein obtained by linking two membrane fusion-related conserved amino acid sequences HR1 and HR2 of the SARS-CoV-2 membrane protein S2 protein by means of a linking peptide. The recombinant fusion protein can be induced and expressed in Escherichia coli, has high expression quantity, and is easy to purify. The SARS-CoV-2 recombinant fusion protein provided by the present invention can form and maintain a stable dimer structure, simulates the conformation of a SARS-CoV-2 membrane fusion intermediate state, can be used as a detection raw material for detecting a SARS-CoV-2 membrane fusion process, has good anti-SARS-CoV-2 activity and good immunogenicity, and has a wide application prospect in the fields of development of drugs for preventing or treating SARS-CoV-2 proteins and development of SARS-CoV-2 vaccines and anti-SARS-CoV-2 antibodies.

## Description

### Technical Field

The present invention relates to a recombinant fusion protein of novel coronavirus, in particular to a recombinant fusion protein derived from HR region of S2 protein of SARS-COV-2 and application thereof.

### Technical Background

SARS-CoV-2 is a single positive-stranded RNA virus with an envelope and linear genome. It belongs to the Orthocoronavirinae subfamily of the Coronaviridae family of the Nidovirales order, and is a novel coronavirus of theβ genus. The novel coronavirus was discovered in 2019, and then caused a pandemic worldwide. It has the characteristics of human-to-human transmission in incubation period, spreads rapidly, and can cause various types of mild, moderate and severe symptoms of lungs, respiratory tract and other organs of infected people. It is a highly pathogenic pathogen. On February 12, 2020, the World Health Organization officially named the disease caused by SARS-CoV-2 as COVID-19.

The genome of novel coronavirus is 26kb-30kb in size and contains multiple open reading frames, which can encode 4 major structural proteins and 16 non-structural proteins. Its main structural proteins are spike protein (S), membrane protein (M), envelope protein (E), and nucleocapsid protein (N). Among them, the spike protein (S) is a trimer and plays an important role in the entry of viruses into host cells. The spike protein monomer is composed of S1 protein and S2 protein. When the virus approaches the host cell, the RBD domain of S1 protein can bind to the ACE2 receptor of the host cell, thereby inducing the process of virus adsorption and entry into the cell. At the same time, S1 protein and S2 protein are cleaved. There are two important functional domains on the S2 protein: HR1 and HR2. These two structural domains then interact with each other to form the six HR helix structures of the HR1-HR2 trimer, further narrowing the distance between the virus membrane and the cell membrane, promoting the fusion of the virus membrane and the cell membrane, and inducing the virus to enter the cell.

At present, the research and development of novel coronavirus protein vaccine and antibody mainly focus on the RBD domain of novel coronavirus S1 protein. There is limited research on the HR1 and HR2 domains on the S2 protein. HR1 and HR2 domains can mediate the fusion of virus and host membrane, and are one of the design targets of drugs and vaccines for novel coronavirus. However, how to simulate the natural conformation before the fusion of HR1 and HR2 domains, inhibit the binding of HR1 and HR2, and block the fusion process of novel coronavirus and cell membrane is the design difficulty of anti-novel coronavirus drugs and novel coronavirus vaccines. No relevant patent or literature has been retrieved yet. The fusion protein disclosed in Application No. CN202011346373.6 "A fusion protein of transmembrane expression of novel coronavirus antigen S2, recombinant vector, recombinant dendritic cell and application thereof' includes sequentially linked CD4 signal peptide, novel coronavirus antigen S2 protein, Flag tag sequence and CD4 transmembrane domain. Compared to the present invention, a completely different strategy was adopted.

### Summary of the invention

The objective of the present invention is to provide a stable recombinant fusion protein derived from HR region of novel coronavirus S2 protein and application thereof.

The technical solution adopted by the present invention is:
A recombinant fusion protein HR121 or HR212 of the HR region of novel coronavirus S2 protein, which is composed of a truncated protein HR1 derived from the HR1 region of the novel coronavirus S2 protein, a truncated protein HR2 derived from the HR2 region, and a linking peptide conjugating the two above. The protein structure of the HR121 is HR1-Linkerl-HR2-linker2-HR1; the protein structure of the HR212 is HR2-Linker2-HR1-linker 1-HR2, wherein the HR1 amino acid sequence is shown in SEQ ID NO. 1, or comprises a sequence that one or more amino acids are substituted, deleted or inserted in the amino acid sequence shown in SEQ ID NO.1. The HR2 amino acid sequence is shown in SEQ ID NO.2 or comprises a sequence that one or more amino acids are substituted, deleted or inserted in the amino acid sequence shown in SEQ ID NO.2, and the amino acid sequence of theLinker1, Linker2 is shown SEQ ID NOs.3-4, respectively.

Further, the amino acid sequence of the novel coronavirus recombinant fusion protein HR121 is shown in SEQ ID NO.5.

Further, the amino acid sequence of the novel coronavirus recombinant fusion protein HR212 is shown in SEQ ID NO.6.

Further, the nucleotide sequence encoding HR121 is shown in SEQ ID NO.7.

Further, the mRNA sequence encoding HR121 is shown in SEQ ID NO.23.

Further, the nucleotide sequence encoding HR212 is shown in SEQ ID NO.8.

Further, the mRNA sequence encoding HR212 is shown in SEQ ID NO.27.

The present invention also protects a vector for expressing the recombinant fusion protein derived from the HR region of novel coronavirus S2 protein, which is characterized in that a HR121 skeleton vector, a HR212 skeleton vector, and the HR121 skeleton vector and HR212 skeleton vector contain the DNA coding sequence of SEQ ID No. 7 and 8, respectively, and the mRNA coding sequence of SEQ ID No. 23 and 27, respectively. In another preferred embodiment, the vector is selected from an mRNA vaccine vector or an adenovirus vector.

The present invention also protects a use of the recombinant fusion protein derived from the HR region of novel coronavirus S2 protein in the manufacture of an antigen detection reagent and a medicament for prevention or treatment of novel coronavirus.

In addition, the present invention also protects a use of the recombinant fusion protein derived from the HR region of novel coronavirus S2 protein as an immunogen in the manufacture of a vaccine and an antibody for novel coronavirus.

Due to the source of the HR region of novel coronavirus S2 protein, the novel coronavirus recombinant proteins HR121 and HR212 provided by the present invention can simulate the intermediate conformation of the membrane fusion of the novel coronavirus and act on the membrane fusion phase of the novel coronavirus. As a new target for inhibiting the replication of novel coronavirus, it is significantly different from the recombinant RBD protein (acting on the adsorption phase of novel coronavirus) reported at present, which is derived from the S1 protein of novel coronavirus. Moreover, these two proteins can induce high affinity neutralizing antibodies against novel coronavirus in animals, and their antibody activity and animal protection effect are equivalent to the antibody activity induced by RBD protein reported at present (Nature. 2020 Oct; 586 (7830): 572-577 doi: 10.1038/s41586-020-2599-8).

In another preferred embodiment, the fusion protein in the present invention can be administered directly or indirectly.

In another preferred embodiment, the direct administration includes administration in the form of recombinant protein vaccines, or the indirect administration includes administration in the form of nucleic acid vaccines (DNA or RNA vaccines), recombinant virus vector vaccines (such as adenovirus vectors, poxvirus vectors, adeno-associated virus vectors, herpes simplex virus vectors, cytomegalovirus vectors), etc.

Compared to the prior art, the beneficial effects of the present invention are:
1. The novel coronavirus recombinant fusion protein provided by the present invention can form and maintain a stable dimer structure, simulate the conformation of the intermediate state of the membrane fusion of novel coronavirus, and can be used as the detection raw material for detecting the membrane fusion process of novel coronavirus.
2. The novel coronavirus recombinant protein provided by the present invention has good anti-novel coronavirus activity, and has broad application prospects in the field of protein drug development for preventing or treating novel coronavirus.
3. The novel coronavirus recombinant protein provided by the present invention has good immunogenicity, can induce high affinity novel coronavirus neutralizing antibodies in animals, and protect novel coronavirus susceptible animals such as golden hamsters from virus infection. It has broad application prospects in the development of novel coronavirus vaccine and anti-novel coronavirus antibody.
4. The novel coronavirus recombinant protein and the induced novel coronavirus neutralizing antibody provided by the present invention have broad-spectrum anti-novel coronavirus performance.

### Description of the drawings

Figure 1 is the structural diagram of the novel coronavirus recombinant fusion proteins HR121 and HR212; Among them, the HR121 fusion protein is formed by connecting HR1-finker1-HR2-linker2-HR1; HR212 is formed by connecting HR2-linker2-HR1-linker1-HR2.
Figure 2 is the SDS-PAGE analysis diagram of the novel coronavirus recombinant fusion proteins HR121 and HR212, in which Figure 2A is the electrophoresis diagram of HR121. M (Marker): 170kDa, 130kDa, 100kDa, 70kDa, 55kDa, 40kDa, 35kDa, 25kDa, 15kDa, 10kDa (from top to bottom); 1: HR121 (20 µg); 2: HR121 (50 µg); 3: HR121 (50 µg); Figure 2B is the electrophoresis diagram of HR212. M (Marker): 170kDa, 130kDa, 100kDa, 70kDa, 55kDa, 40kDa, 35kDa, 25kDa, 15kDa, 10kDa (from top to bottom); 1: HR212 (10 µg); 2: HR212 (20 µg); 3: HR212 (50 µg).
Figure 3 is the secondary structure diagram of the novel coronavirus recombinant fusion proteins HR121 and HR212 by circular dichroism analysis. Among them, Figure 3A shows the structure diagram that HR121 can form α Spiral; Figure 3B shows the structure diagram that HR212 can form α Spiral.
Figure 4 is the activity diagram of the novel coronavirus recombinant fusion proteins HR121 and HR212 against novel coronavirus.
Figure 5 is the antibody titer diagram of rabbits immunized with the novel coronavirus recombinant fusion proteins HR121 and HR212.
Figure 6 shows that both the rabbit antiserum against the novel coronavirus recombinant fusion proteins HR121 and HR212 can effectively inhibit the entry of novel coronavirus novel coronavirus into 293T-ACE2 cells.
Figure 7 shows the activity diagram of the rabbit serum against novel coronavirus after immunizing rabbits with the novel coronavirus recombinant proteins HR121 and HR212.
Figure 8 shows the activity diagram of the rabbit antibody against novel coronavirus after immunizing rabbits with the novel coronavirus recombinant proteins HR121 and HR212.
Figure 9 shows that after immunizing hamsters with novel coronavirus recombinant protein HR121, the novel coronavirus infection of hamster lung tissue can be effectively inhibited.
Figure 10 is the sequence comparison diagram of the other three HR121 and three HR212 of novel coronavirus with different amino acids being deleted in the HR1 and HR2 regions.
Figure 11 is the SDS-PAGE electrophoresis of the other three HR121 and three HR212 recombinant proteins with different amino acid sequences being deleted in the HR1 and HR2 regions of novel coronavirus; M: Marker: 100kDa, 70kDa, 40kDa, 25kDa, 20kDa, 15kDa, 10kDa (from top to bottom); 1: HR121-1 (20 µg); 2: HR121-2 (20 µg); 3: HR121-3 (20 µg); 4: HR212-1 (20 µg); 5: HR212-2 (20 µ g) ; 6: HR212-3 (20 µg).
Figure 12 shows that the other three HR121 and three HR212 recombinant proteins with different amino acid sequences being deleted in the HR1 and HR2 regions of novel coronavirus also have inhibitory activity against novel coronavirus.
Figure 13 shows that the rabbit antiserum against the novel coronavirus recombinant protein HR121 effectively inhibits the entry of 23 strains of novel coronavirus mutant pseudoviruses into 293T-ACE2 cells.
Figure 14 shows that after immunizing hamsters with the novel coronavirus recombinant protein HR121, novel coronavirus Omicron BA.2 mutant infection of hamster lung tissue can be effectively inhibited.

### Detailed description

Through extensive and intensive research, the inventor designed a class of recombinant proteins based on the HR region of the novel coronavirus S2 protein (such as the novel coronavirus recombinant proteins HR121 and HR212), which can simulate the intermediate conformation of membrane fusion of novel coronavirus and block the fusion process between the virus and the cell membrane. The fusion protein of the present invention has good immunogenicity, can induce high affinity neutralizing antibodies in animals, and has excellent anti-novel coronavirus activity. Therefore, it can be used for drug development related to novel coronavirus. On this basis, the present invention has been completed.

Preferably, the neutralizing antibody induced by the fusion protein of the present invention has broad-spectrum anti-novel coronavirus performance. At the cellular level, the neutralizing antibody induced by the fusion protein has the activity of anti-many kinds of SARS-CoV-2 mutant strain viruses entering the cells, and can protect the susceptible animals of novel coronavirus from the infection of virus mutant strains (such as Omicron BA.2). Preferably, the SARS-CoV-2 mutant strain virus is selected from the group consisting of: S477N, E484K, A222V, N439K, K417N, D614G, D839Y, B.1.617, B.1.617.1, B.1.617.2.V2, B.1.429, B.1.525, B.1.526, B.1.1.7, B.1.351, B.1.1.28, B.1.617.2, C.37, B.1.621, B.1.1.529 (Omicron BA.1), Omicron BA.2, Omicron BA.3, and Omicron BA.4/5 of pCDNA3.1-SARS-CoV-2 V-2-Spike- Δ 18.

### Terms

In order to better understand this disclosure, certain terms are first defined. As used in this application, each of the following terms shall have the meaning given below unless expressly provided herein. Other definitions are elaborated throughout the entire application.

The term "about" may mean a value or composition within the acceptable margin of error of a particular value or composition as determined by a general technician in the field, which will depend in part on how the value or composition is measured or determined. For example, as used herein, the expression "about 100" comprises all values between 99 and 101 (e.g., 99.1, 99.2, 99.3, 99.4, etc.).

As used herein, the term "containing" or "comprising (including)" may be open, semi-closed, and closed. In other words, the term also includes "essentially consisting of" or "consisting of".

As used herein, the term "effective dose" typically refers to the amount of immunogen that can induce a protective immune response sufficient to induce immunity to prevent and/or alleviate infection or disease and/or reduce at least one symptom of infection or disease.

As used herein, the term "protective immune response" or "protective response" refers to an immune response mediated by an immunogen against an infectious agent or disease, which is demonstrated in vertebrates (such as humans) to prevent or alleviate infection or reduce at least one disease symptom.

### The novel coronavirus recombinant fusion protein of the present invention

In the present invention, "fusion protein of the present invention", "antigen protein of the present invention", "immunogenic peptide of the present invention", "recombinant fusion protein of the present invention", "novel coronavirus recombinant fusion protein of the present invention" and "novel coronavirus recombinant protein of the present invention" can be used interchangeably, all refer to a recombinant fusion protein derived from the HR region of the novel coronavirus S2 protein. The fusion protein of the present invention comprises: HR1 or HR2 amino acid sequences or sequences with substitution (such as conservative amino acid substitution), deletion, or insertion of one or more amino acids. It should be understood that the term includes not only one kind of fusion protein of the present invention, but also a protein set (or polypeptide set) formed by multiple fusion proteins of the present invention.

Preferably, the fusion protein of the present invention comprises a structure as shown in the formulas below:
HR1-Linker1-HR2-Linker2-HR1 (Formula I),
HR2-Linker2-HR1-Linker 1-HR2 (Formula II);
wherein, HR1 is a truncated protein of the HR1 region or a sequence thereof, in which one or more amino acids are substituted, deleted, or inserted;
HR2 is a truncated protein of the HR2 region or a sequence thereof, in which one or more amino acids are substituted, deleted, or inserted;
the amino acid sequences of Linker1 and Linker2 are shown in SEQ ID NO.3 or 4.

In another preferred embodiment, the truncated protein of the HR1 region or the sequence thereof in which one or more amino acids are substituted, deleted, or inserted, includes:
(1) The amino acid sequence as shown in SEQ ID NO.1;
(2) Compared to the amino acid sequence of HR1 (SEQ ID NO.1), an amino acid sequence in which one or more amino acids (such as 5-10 amino acids, 7-12 amino acids, 9-14 amino acids, 11-16 amino acids, or 13-18 amino acids) are deleted from the N-terminus; and/or one or more amino acids (such as 10-15 amino acids, 14-19 amino acids, 18-23 amino acids, 20-25 amino acids, 22-27 amino acids, 24-29 amino acids, 28-31 amino acids, or 30-35 amino acids) are deleted from the C-terminus.

Preferably, the amino acid sequence of HR1 is selected from the group consisting of:
(1) SEQ ID NO. 1 (HR1);
(2) SEQ ID NO. 11 (HR1- ①;
(3) SEQ ID NO. 12 (HR1- ②; or
(4) SEQ ID NO. 13 (HR1- ③.

In another preferred embodiment, the truncated protein of the HR2 region or the sequence thereof in which one or more amino acids are substituted, deleted, or inserted, includes:
(1) The amino acid sequence shown in SEQ ID NO. 2;
(2) Compared to the amino acid sequence of HR2 (SEQ ID NO.2), an amino acid sequence in which one or more amino acids (e.g., 1, 2, 3, 4, 5, 6, 7, 8, 9, 11, 12 amino acids) are deleted from the N-terminus; and/or one or more amino acids (e.g., 1, 2, 3, 4, 5, 6, 7, 8 amino acids) are deleted from the C-terminus.

Preferably, the amino acid sequence of HR2 is selected from the group consisting of:
(1) SEQ ID NO. 2 (HR2);
(2) SEQ ID NO. 14 (HR2- ①;
(3) SEQ ID NO. 15 (HR2- ②; or
(4) SEQ ID NO. 16 (HR2- ③.

Preferably, the amino acid sequence of the novel coronavirus recombinant fusion protein (HR121) is shown in SEQ ID NO.5; or the amino acid sequence of the novel coronavirus recombinant fusion protein (HR212) is shown in SEQ ID NO.6.

Preferably, the novel coronavirus recombinant fusion protein further includes an amino acid sequence selected from the group consisting of:
(1) HR121-1: Connected by HR1- ①- Linker1- HR2- ①- Linker2- HR1- ①;
(2) HR121-2: Connected by HR1- ②Linker1- HR2- ②Linker2- HR1- ②;
(3) HR121-3: Connected by HR1- ③- Linker1- HR2- ③- Linker2- HR1- ③;
(4) HR212-1: Connected by HR2 ①- Linker2 HR1 ①- Linker1 HR2- ①;
(5) HR212-2: Connected by HR2- ②Linker1-HR1- ②Linker2-HR2- ②;
(6) HR212-3: Connected by HR2- ③Linker1 HR1- ③Linker2 HR2- ③.

Preferably, the conservative amino acid substitution is carried out according to Table A.

**Table A**

| The initial residue | Representative substitutions | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (L) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

In the present invention, the fusion protein also includes other forms, such as pharmaceutically acceptable salts or conjugates.

As used herein, the protein set described by the term "protein set" consists of at least two fusion proteins of the present invention or derived sequences thereof (substitutions, deletion, or insertion).

In addition, the protein set may further include other coronavirus SARS-CoV-2 antigenic peptides or proteins besides SEQ ID NO.: 5 and 6.

As used herein, the term "isolated" refers to the isolation of a substance from its original environment (if it is a natural substance, the original environment is the natural environment). For example, the protein of living cells in the natural state is not isolated and purified, but the same protein is isolated and purified if it is isolated from other substances that exist in the natural state.

The fusion protein of the present invention can be a recombinant protein or a synthetic protein, preferably a synthetic protein.

In the present invention, when the sequence of the fusion protein is short (e.g., ≤ 70aa, more preferably ≤ 60aa), the relevant peptide sequence can be directly synthesized using chemical methods.

When the sequence of the fusion protein of the present invention is long or the fusion protein of the present invention is provided in the form of a fusion protein, the relevant peptide sequences can also be obtained in large quantities using recombination method. This usually involves cloning the coding sequence of the antigen polypeptide or its fusion protein into a vector, followed by transferring it into cells, and then isolating the relevant antigen polypeptide or fusion protein from the proliferated host cells by conventional methods.

Through the provided sequence information, skilled technicians can use the available cloning technology to generate nucleic acid sequences or vectors suitable for being transduced into the cells.

The vector can be a plasmid or a virus. The viral vector has the ability to enter cells. However, non-viral vectors such as plasmids can be combined with reagents to facilitate the uptake of viral vectors by target cells. Such reagents include polycation agents. Optionally, the delivery system, such as a liposome-based delivery system, can be used. The vector used in the present invention is preferably suitable for use *in vivo* or *in vitro.*

The vector will preferably contain one or more regulating sequences to guide the expression of nucleic acid sequences in target cell. The regulating sequence may include a promoter, an enhancer, a transcriptional termination signal, a polyadenylation sequence, an origin of replication, a nucleic acid restriction, and homologous recombination site that can be operably linked to the nucleic acid sequence. The vector may also include selective marks, such as for determining the expression of the vector in the growth system (such as a bacterial cell) or in the target cell.

### Pharmaceutical composition

The present invention also provides a pharmaceutical composition. The pharmaceutical composition of the present invention can be therapeutic or preventive (such as vaccines, vaccine compositions, or immunogenic compositions). The pharmaceutical composition of the present invention includes an effective amount of the fusion protein or protein sets, nucleotide molecules, vectors, and/or vaccines for host cells of the present invention, or immune cells activated by the fusion protein of the present invention (such as dendritic cells sensitized with the fusion protein of the present invention or T cells induced by dendritic cells), as well as at least one pharmaceutically acceptable carrier, diluent, or excipient.

In the present invention, these drug compositions contain immune antigens (including the fusion protein, peptide set or derivatives thereof of the present invention), and are usually combined with "pharmaceutically acceptable carriers", including any carrier that itself does not induce the production of antibodies that are harmful to the individual receiving the composition. Examples of suitable carriers include (but are not limited to) proteins, lipid aggregates (such as oil droplets or liposomes) and so on. These carriers are well known to those of ordinary skill in the art. In addition, these carriers may function as immunostimulants ("adjuvants").

Preferably, the carrier is a pharmaceutically acceptable carrier. These carriers include any carrier that does not induce the production of harmful antibodies to individuals receiving the composition itself. In a preferred embodiment, the pharmaceutically acceptable carrier comprises liquid, preferably water, saline or buffer. The carrier may further contain an auxiliary substance, preferably a filler, a lubricant, a glidant, a wetting agent or an emulsifier, a pH buffer substance, etc.

In addition, the pharmaceutical composition of the present invention may further contain additional adjuvants, such as vaccine adjuvants. Representative vaccine adjuvants include (but are not limited to) the following types: inorganic adjuvants, such as aluminum hydroxide, alum, etc.; synthetic adjuvants, such as synthetic double-stranded polynucleotides (double-stranded polyadenosine acid, polyuridine acid), levamisole, isopinosine, etc.; oil agents, such as Freund's adjuvant, peanut oil emulsification adjuvant, mineral oil, vegetable oil, etc.. Examples of known adjuvants include but are not limited to: complete Freund's adjuvant, incomplete Freund's adjuvant, aluminum hydroxide adjuvant, lipopolysaccharide (LPS), RIBI adjuvant, MF-59, SAS adjuvant, MF59 adjuvant, QS-21 adjuvant, Poly I: C and other TLR ligands, GM-CSF, IL-2, IL-3, IL-7, IL-11, IL-12, IL-18, IL-21, etc.

Generally, the pharmaceutical composition (vaccine composition or immunogenic composition) may be made into an injectable agent, for example, a liquid solution or suspension; it may also be made into a solid form suitable for being formulated into a solution or suspension or a liquid excipient before injection. The preparation may also be emulsified or encapsulated in liposomes to enhance the adjuvant effect.

In another preferred embodiment, the pharmaceutical composition may further contain a vaccine component derived from one or more pathogens selected from the group consisting of: other viruses, and combinations thereof. The vaccine component comprises an inactivated strain, an attenuated strain, or a protein, a nucleic acid, etc.

The prepared pharmaceutical combination can be administered through conventional routes, including (but not limited to) muscle inoculation, intradermal inoculation, subcutaneous inoculation, nasal drip, nebulized inhalation, reproductive tract, rectum, oral administration, or any combination thereof.

When using a pharmaceutical composition, a safe and effective amount of the fusion protein or peptide set of the present invention is administered to a human, wherein the safe and effective amount is usually at least about 1 ug peptides/kg body weight, and in most cases not more than about 8 mg peptides/kg body weight, preferably the dose is about 1 ug-1 mg peptides/kg body weight. Of course, the particular dose should also depend on various factors, such as the route of administration, patient's healthy status, which are well within the skills of an experienced physician.

### Application

It should be understood that the fusion protein in the present invention can be administered directly in the form of a protein (such as a recombinant protein vaccine) or indirectly. For example, said indirect administration includes common measns in this field. For example, it is administered to a subject in need thereof in the form of a nucleic acid vaccine (DNA or RNA vaccine), a recombinant viral vector vaccine (such as an adenovirus vector, a poxvirus vector, an adeno-associated virus vector, a herpes simplex virus vector, a cytomegalovirus vector), etc.. The preferred vector vaccine is an adeno-associated virus vector vaccine.

In some embodiments, the vaccine is a nucleic acid vaccine (DNA or RNA vaccine), a recombinant protein subunit vaccine, a recombinant viral vector vaccine, a recombinant bacterial vector vaccine, a virus like particle vaccine, a nanoparticle vaccine, and a cell vector vaccine.

Preferably, the vaccine of the present invention can be a recombinant protein vaccine, a recombinant RNA vaccine, a recombinant viral vector vaccine (such as an adenovirus vector, a poxvirus vector, an adeno-associated virus vector, a herpes simplex virus vector, a cytomegalovirus vector), a recombinant bacterial vector vaccine, a recombinant yeast vector vaccine, or a recombinant virus like particle vaccine.

In some embodiments, the vaccine of the present invention is selected from a recombinant protein vaccine, a recombinant RNA vaccine, a recombinant adenovirus vector, a recombinant poxvirus vector, or a combination of one or two or more of them.

In some embodiments, one or more of the vaccines are used for vaccination, such as co-vaccination or sequential vaccination.

In some embodiments, one or more of the vaccines and other vaccines against novel coronavirus are used for vaccination, for example, the other vaccines include vaccines against coronavirus S or S1, for example, the S or S1 is derived from, including but not limited to, SARS CoV-2, SARS-CoV, MERS-CoV, HCoV-229E, HCoV-OC43, HCoV-NL63, HCoV-HKU1, bat-CoV, etc.

In some embodiments, the vaccine is a recombinant protein vaccine. In some embodiments, the vaccine comprises a combination of a nucleic acid vaccine (DNA or RNA vaccine) and a recombinant human-derived cell vector vaccine.

In one aspect of the present invention, a method for preparing a vaccine against novel coronavirus SARS-CoV-2 is provided, which comprises steps of:
(a) Providing the fusion protein, nucleotide molecule, vector, and/or host cell of the present invention;
(b) Combining the active substance provided in (a) with an immunologically or pharmaceutically acceptable carrier.

The vaccine of the present invention can be used to induce an immune-enhancing protective immune response to prevent and/or mitigate related diseases caused by the novel coronavirus SARS-CoV-2.

In another preferred embodiment, the related disease caused by novel coronavirus SARS-CoV-2 is selected from group consisting of: respiratory tract infection, pneumonia and its complications, and a combination thereof.

Preferably, the vaccine herein can prevent, eliminate or alleviate the infection of novel coronavirus or at least one symptom thereof, such as respiratory symptoms (such as nasal congestion, sore throat, hoarseness), headache, cough, sputum, fever, rale, wheezing, dyspnea, pneumonia caused by infection, severe acute respiratory syndrome, renal failure, etc.

In one aspect of the present invention, it also provides a method for preventing or treating novel coronavirus SARS CoV-2, which comprises a step of giving the fusion protein, nucleotide molecule, vector, host cell and/or vaccine of the present invention to a subject in need thereof.

The following will further illustrate the technical solution of the present invention through experiments.

### I. Materials and reagents:

1. Main materials
   Virus: novel coronavirus (SARS-CoV-2) clinical isolated strain (National Microbiology Data Center, serial number: NMDCN0000HUI); Novel coronavirus Omicron BA.2 clinical isolated strain (National Microbiology Data Center, serial number: UB1663744906574);
   Novel coronavirus pseudovirus (The pseudovirus was obtained by transfection of 293FT cells with VSVΔG-*G and pcDNA3.1-SARS-CoV-2-envΔ18);
   Plasmid amplification strain: E.coli DH5 α competent state; Protein expression strain: E.coli BL21 (DE3) competent state (Beijing Novozan).
2. Main reagents
   LB medium, 2XYT medium (Sigma);
   DMEM medium, FBS (Gibco);
   JetPRIME transfection reagent (polyplus transfer, Illkirch, France)
   Kanamycin (Sigma Aldrich, St Louis, USA)
   Endotoxin-free Small Plasmid Extraction Kit, Large Plasmid Extraction Kit (Tiangen, Beijing)
   TaKaRa BCA Protein Assay Kit (TaKaRa Bio, Beijing)
   CCK8 reagent kit (Beyotime, Shanghai)
   Roche viral RNA Extraction Kit (Roche Diagnostics, Mannheim, Germany)
   Renilla Luciferase Kit (Promega, Madison, USA)
   Trizol reagent (Invitrogen, CA, USA)
   Protein A Filler Purification Kit (BBI life science, Shanghai)
   Complete and incomplete Freund's adjuvant (Sigma Aldrich, USA)
   Aluminum Adjuvant (Alhydrogel^{®} adjuvant 2%, Invivogen, USA)

### 2. Examples

### Example 1: Construction of novel coronavirus SARS-CoV-2 recombinant protein expression vectors pET-30a-SARS-CoV-2-HR121 and pET-30a-SARS-CoV-2-HR212

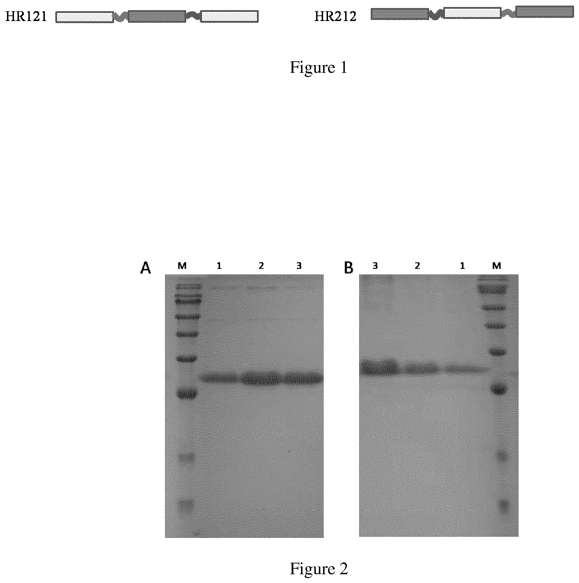

1.1 Construction of pET-30a-SARS-CoV-2-HR121 vector:
   The nucleotide sequence (SEQ ID NO. 7) of novel coronavirus HR121 was genetically synthesized (Shanghai Jierui Biological Engineering Co., Ltd.) and inserted between the EcoRI (GAATTC) and XhoI (CTCGAG) digestion sites of pET-30a vector.
   The synthesized pET-30a-SARS-CoV-2-HR121 vector was transformed into competent E.coli DH5α. After cultured at 37°C for 12 h in a liquid LB medium containing 50 µg/mL kanamycin, a small amount of plasmid was extracted for furture use.
1.2 Construction of pET-30a-SARS-CoV-2-HR212 vector:
   The nucleotide sequence (SEQ ID NO. 8) of novel coronavirus HR212 was genetically synthesized (Shanghai Jierui Biological Engineering Co., Ltd.) and inserted between the EcoRI (GAATTC) and XhoI (CTCGAG) digestion sites of pET-30a vector
   The synthesized pET-30a-SARS-CoV-2-HR212 vector was transformed into competent E.coli DH5α, and cultured in a liquid LB medium containing 50 µg/mL kanamycin at 37°C for 12 h, and a small amount of plasmid was extracted for furture use.
   The structures of HR121 and HR212 are shown in Figure 1.

### Example 2: Induced expression and purification of recombinant proteins HR121 and HR212 of novel coronavirus SARS-CoV-2

2.1. The plasmid was transformed into the competent BL21(DE3) cells by pET-30a-SARS-CoV-2-HR121 or pET-30a-SARS-CoV-2-HR212 plasmid according to the conventional transformation method;
2.2. Single colony was selected, and cultured and shaken in a 2×YT medium containing 5 mL kan at 37 ° C until cloudy. 100 µL of bacteria solution was inoculated into 100 mL of 2×YT medium at 180rpm, 37 ° C for a total of 500 mL and shaken overnight (12h).
2.3 0.238 g of IPTG was weighed and dissolved in 1 mL of water, 100 µL IPTG solution was added to every 100 mL of water, shaken overnight at 140 rpm at 20 °C (12 hours);
2.4 Bacterial solution was collected, resuspended in a 20 mM imidazole solution, and resuspended in 5mL 20mM imidazole solution for every 100mL of bacterial solution;
2.5 subjected to Ultrasound fragmentation, stopped for 1 second after 1 second, 216000 Joules. During ultrasound, pay attention to ice bath to prevent high temperature from damaging proteins. Ultrasonification casued the cloudy bacterial liquid slightly transparent (about 10-20 minutes of ultrasonification);
2.6 10% Tritonx100 was added to the ultrasound-treated bacterial solution until the final concentration reached 1%, and was placed on ice for 30 minutes;
2.7 The solution was centrifuged at 12000 rpm for 30 minutes, and the supernatant was poured into a new centrifuge tube, the process was repeated, and then the supernatant was poured into the new centrifuge tube;
2.8 The sample was loaded on a Nickel column (GE Ni SepharoseTM6 Fast Flow; Item No.: 17-5318-06), before loading the column, the column was washed with 10 times of the volume of 20 mM imidazole solution, and after washing, the column was loaded;
2.9 After being loaded with the sample, the colunm was wash with 20 times of the column volume (40 mL) of 20 mM imidazole solution to remove impurities;
2.10 The column volume (10 mL) was washed with 5 times of 100 mM imidazole solution to remove impurities;
2.11 After washing, the target protein was eluted with 25 times of the column volume (50 mL) of 200 mM imidazole solution;
2.12 The target protein was concentrated in batches using a 15 mL 10KD concentration tube. After the sample was loaded, the 200 mM imidazole solution was replaced with PBS solution. That is, after the sample was concentrated, PBS solution was added to the concentration tube, and the tube was centrifuged to replace the 200 mM imidazole solution. This process was repeated for three times to ensure that the liquid for dissolving the protein was PBS;
2.13 Approximately 1.5 mL of the concentrated protein solution was inhaled into a 1.5 mL EP tube and the protein concentration was quantified using a BCA assay kit. The solution was divided into small tubes to avoid repeated freeze-thaw cycles.

### Example 3: SDS-PAGE protein electrophoresis analysis of novel coronavirus SARS-CoV-2 recombinant proteins HR121 and HR212

3.1 The purified protein was quantified using BCA assay kit.

3.2 50 µg HR121 and HR212 proteins were taken and separated by electrophoresis on 10% SDS-PAGE gel. After electrophoresis, Coomassie brilliant blue method was used for staining and decolorization. The analysis and detection results are shown in Figure 2. The size of HR121 protein is about 28kDa, and the size of HR212 protein is about 24kDa.

### Example 4: Secondary structure analysis of recombinant proteins HR121 and HR212 of novel coronavirus by circular dichroic chromatography

4.1. Expression vector construction and protein purification of recombinant HR1 and HR2 of novel coronavirus: The novel coronavirus HR1 nucleotide coding sequence (SEQ ID NO.9) and the novel coronavirus HR2 nucleotide coding sequence (SEQ ID NO.10) were inserted between the EcoRI(GAATTC) and XhoI (CTCGAG) restriction sites of pET-30a vector, respectively. Recombinant proteins HR1 and HR2 of novel coronavirus were obtained by protein purification according to the method of Example 2.

4.2 Jasco spectrophotometer (model J-815) was used to detect the light absorption of 1 µ M HR121,1 µ M HR121+1 µ MHR2, 1 µ M HR212, 1 µ M HR212+1 µM HR2. The binding ability of HR121 protein and HR2 protein was detected; The binding ability of HR212 protein and HR1 protein was detected.

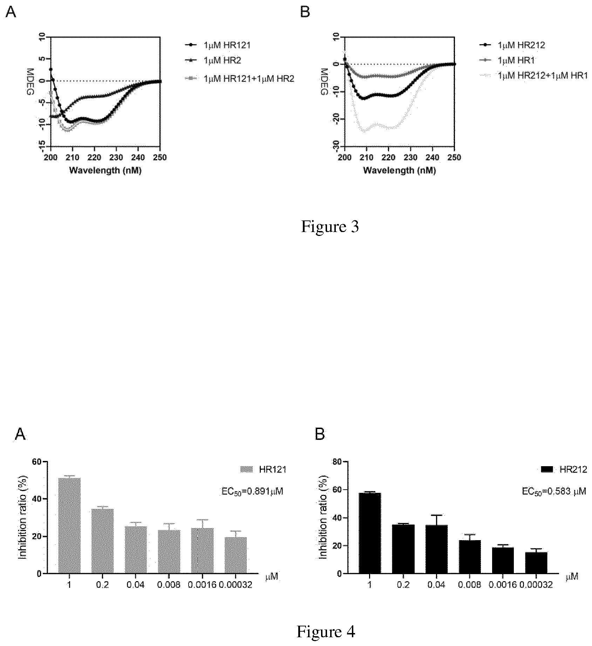

The analysis and detection results are shown in Figure 3. HR121 protein can bind to HR2 protein, forming a stable α Spiral structure. HR212 protein can bind with HR1 protein to form a stable α Spiral structure. Therefore, both of two proteins can be used to prepare novel coronavirus antigen detection reagents.

### Example 5: Detection of anti-novel coronavirus activity of recombinant proteins HR121 and HR212 of novel coronavirus

The antiviral activity of cells was detected by CCK8 method.

5.1. Vero E6 cells were inoculated with 100 µl of 5×10⁵ /mL Vero E6 cells per well on a 96-well cell culture plate and cultured overnight at 37°C in a CO2 incubator.

5.2. Then, 50 µl of SARS-CoV-2 virus at MOI of 0.1 was added to each well to infect the cells, and 50 µl of the novel coronavirus recombinant proteins HR121 and HR212 were added to each well. After incubation for 2 h, the medium was replaced with 200 µl fresh media containing the novel coronavirus recombinant fusion proteins HR121 and HR212 diluted in gradient.

5.3 After 72 hours of cultivation at 37 °C in a CO₂ incubator, CCK8 reagent was added to the culture supernatant, and the OD450 value was read on an enzyme-linked immunosorbent assay (ELISA) reader to quantitatively analyze the cytopathic effects caused by SARS-CoV-2 and the protective effects of HR121 and HR212 on cellular lesions.

The analysis and detection results are shown in Figure 4. It can be seen from Figure 4 (A) that HR121 can inhibit the Vero-E6 cytopathic effect induced by novel coronavirus, and the EC₅₀ of HR121 protein to protect the cytopathic effect caused by SARS-CoV-2 is 0.891 µM; Figure 4(B) shows that HR212 can inhibit Vero-E6 cytopathic effect induced by novel coronavirus, and the EC₅₀ of HR212 protein protecting SARS CoV-2 induced cytopathic effect is 0.583 µM. This indicates that both of two fusion proteins have good anti SARS-CoV-2 activities.

### Example 6 Novel coronavirus recombinant proteins HR121 and HR212 immunize adult New Zealand white rabbits

6.1. Primary immunization: The novel coronavirus recombinant proteins HR121 or HR212 prepared in Example 3 were diluted in PBS solution to a protein concentration of 200 µg/mL. Equal volume of Freund complete adjuvant was added, mixed with protein solution to form a white latex, and each rabbit was immunized with 1 ml via subcutaneous multipoint (protein content was 100 µg/ rabbit).

6.2. Boost immunity: two booster immunizations were performed on 21 and 42 days. The novel coronavirus recombinant proteins HR121 or HR212 prepared in Example 3 were diluted with PBS solution to a protein concentration of 300 µg/mL Equal volume of Freund imcomplete adjuvant was added, mixed with protein solution to form a white latex, and each rabbit was immunized with 1 ml via subcutaneous multipoint (protein content was 150 µg/ rabbit). Simultaneously, a negative control immune group without protein was set up.

6.3 On the 56^{th} day, heart blood was collected from immunized rabbits, serum was collected, and packaged and stored at -80 °C.

### Example 7: Determination of serum antibody titers of recombinant HR121 and HR212 of novel coronavirus in rabbits

7.1 1 µg/ml HR121 or HR212 protein was dissolved in ELISA coating-buffer, coated at 100µl/ well and incubated at 4°C overnight, or at 37°C for 2 hours;
7.2 the plate was washed by using ELISA washing buffer for 3 times, 3 minutes/time, 100 µl of PBS-5% skim milk powder was added to each well, and the plate was sealed at 37°C for 2 hours;
7.3 The plate was washed as above, 100 µl Diluted serum was added to each well, 37 °C/2 hours;
7.4 The plate was washed as above and 100% of 1:5000 diluted sheep anti rabbit IgG-HRP (or anti mouse IgG-HRP) was added at 37 °C for 1 hour; the plate was washed as above and OPD substrate reaction solution was added. After 10 minutes, the reaction was quenched with 2 M sulfuric acid;
7.5 The OD490/630nm value was measured using an ELISA instrument.
7.6 According to the reading value of ELISA, the OD value of the sample > 0.12+negative control OD value is positive.

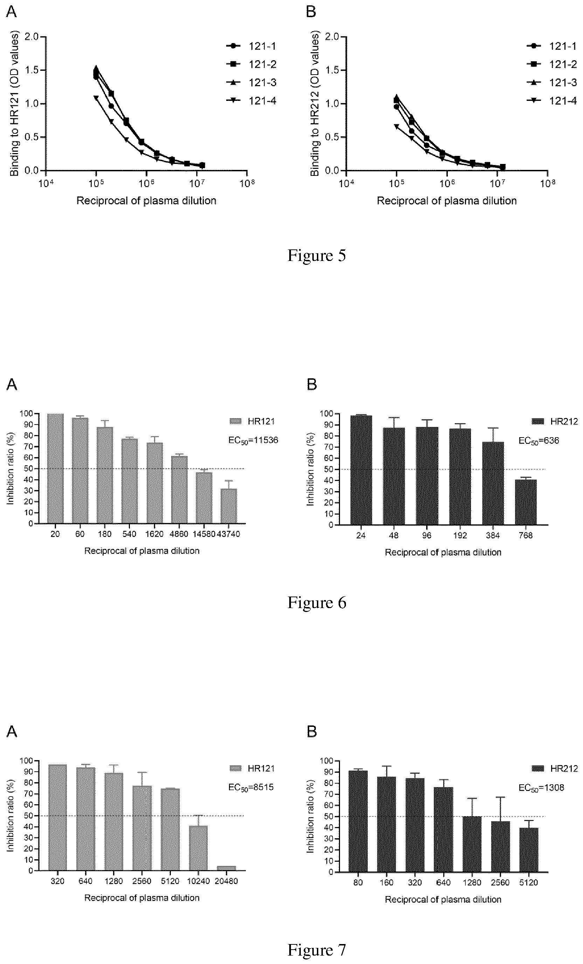

The analysis and detection results are shown in Figure 5, and the anti rabbit serum titers of HR121 and HR212 proteins are both between 10⁶ and 10⁷. This indicates that both proteins can induce high titers of antibodies in animal bodies.

### Example 8: Determination of titer of anti SARS CoV-2 pseudovirus antibody neutralized by rabbit antiserum of recombinant protein HR121 and HR212 of novel coronavirus.

### (1) Determination of titers of pseudovirus antibodies against the original strain of SARS CoV-2 neutralized by rabbit antiserum against recombinant proteins HR121 and HR212 of novel coronavirus

8.1 Preparation and virus titration of pseudovirus of original novel coronavirus strain: according to the operation instructions of the transfection reagent jetPRIME, 10 µg pCDNA3. 1-SARS-CoV-2- Spike- Δ 18 plasmids and 500 µL jetPRIMEBuffer was mixed; 20 µL jetPRIMEBuffer was added; mixed and kept at room temperature for 10 min. The mixture was added dropwise to the 239T cells in a T75 flask, and the culture medium was discarded after 12 h of culture at 37°C and 5% CO₂, and the cells were infected with VSVΔG-VSVG pseudovirus at MOI= 1, washed for 3 times with PBS 6 h after infection, changed to fresh DMEM medium and continued to culture, and the SARS-CoV-2 pseudovirus-containing supernatant was harvested after 24 and 48 hrs. The cells were centrifuged at 1000 rpm/min for 10 minutes and the supernatant was collected, which was then packaged and stored at -80 °C. And virus titer was measured using 239T-ACE2 cells.

8.2 Assay of HR121 and HR212 rabbit serum for neutralization of SARS-CoV-2 pseudovirus: 100 µl of 239T-ACE2 (2×10⁵ cells/ml) per well was incubated for 12 h on a 96-well cell culture plate, 50 µl of serial dilutions of HR121 or HR212 anti-rabbit serum solution was incubated on another 96-well cell culture plate (triplicate wells per dilution), and 50 µl SARS-CoV-2 pseudovirus at an MOI of 0.1 was added per well. Simultaneously positive control wells without protein and negative control wells without virus were set. After incubated at 37 °C for 1 hour, and the mixture of virus and protein was added to the cells. After 48 hours of cultivation at 37 °C in a CO₂ incubator, the fluorescence expression intensity of SARS-CoV-2 pseudovirus was detected using Renilla Luciferase assay kit, and the EC₅₀ value of HR121 or HR212 anti rabbit serum was calculated.

The analysis and detection results are shown in Figure 6, the EC₅₀ value of HR121 protein rabbit antiserum neutralizing SARS-CoV-2 pseudovirus is 11536 times of serum dilution, and the EC₅₀ value of rabbit antiserum neutralizing SARS-CoV-2 pseudovirus with HR212 protein is 636 times of serum dilution. This indicates that the antibodies against these two proteins may have good activities against SARS-CoV-2 virus entering into cells.

### (2) Determination of titers of pseudovirus antibodies against various mutants of SARS CoV-2 neutralized by rabbit antiserum of recombinant protein HR121 of novel coronavirus

8.3 Preparation and virus titration of pseudoviruses of various mutants of novel coronavirus: according to the operation instructions of the transfection reagent jetPRIME, 10 µg of pCDNA3.1-SARS-CoV-2-Spike-Δ18 plasmids containing 23 novel coronavirus mutants: S477N, E484K, A222V, N439K, K417N, D614G, D839Y, B.1.617, B.1.617.1, B.1.617.2.V2, B.1.429, B.1.525, B.1.526, B.1.1.7, B.1.351, B.1.1.28, B.1.617.2, C.37, B.1.621, B.1.1.529 (Omicron BA. 1), Omicron BA. 2, Omicron BA.3, and Omicron BA.4/5, was mixed with 500 µL jetPRIMEBuffer well, respectively, and 20 µL of JetPRIMERegent was added and mixed, and kept at room temperature for 10 minutes. The mixture was dropwise added to 239T cells in a T75 culture bottle. The cells were incubated at 37 °C and 5% CO₂ for 12 hours. The culture was discarded and VSV at MOI = 1 Δ G-VSVG pseudovirus was added for infection, the cells were washed for 3 times with PBS 6 hours after infection, then the medium was replaced with a fresh DMEM medium for further culture, and supernatants containing various SARS-CoV-2 pseudoviruses were harvested 24 and 48 hours later. The cells were centrifuged at 1000 rpm/min for 10 minutes and the supernatant was collected, which was then packaged and stored at -80 °C. And virus titer was measured using 239T-ACE2 cells.

8.4 Assay of HR121 rabbit serum for the neutralization of SARS-CoV-2 pseudovirus: 100 µl of 239T-ACE2 (2×10⁵ cells/ml) per well was added to a 96-well cell culture plate for 12 h, and 50 µl of serial dilution of HR121 anti-rabbit serum solution was added to another 96-well cell culture plate (triplicate wells per dilution), and 50 µl of SARS-CoV-2 pseudovirus at an MOI of 0.1 was added per well. Simultaneously positive control wells without protein and negative control wells without virus were set. After incubation at 37 °C for 1 hour, and the mixture of virus and protein was added to the cells. After incubation at 37°C for 48 h in a CO₂ incubator, the fluorescence expression intensity of SARS-CoV-2 pseudovirus was detected with RenillaLuciferase kit, and the EC₅₀ value of HR121 anti-rabbit serum was calculated.

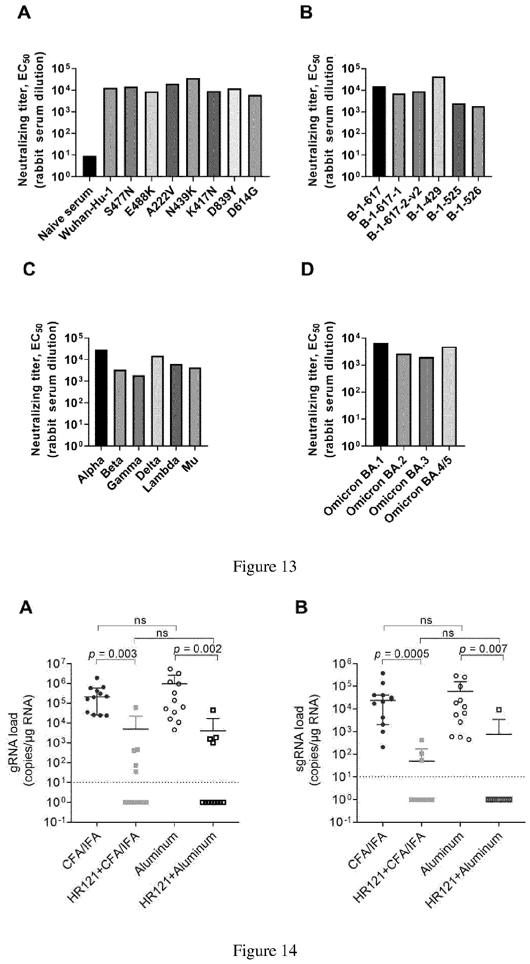

The analysis and detection results are shown in Figure 13. The EC₅₀ values of HR121 protein rabbit serum neutralizing various SARS-CoV-2 pseudoviruses are as follows: 14663 times serum dilution for S477N, 8824 times serum dilution for E484K, 36164 times serum dilution for A222V, 20174 times serum dilution for N439K, 9025 times serum dilution for K417N, 5986 times serum dilution for D614G, 12087 times serum dilution for D839Y, 15199 times serum dilution for B.1.617.1, 7132 times serum dilution for B.1.617.2, and 9023 times serum dilution for B.1.617.2 V2. 433390 times serum dilution for B.1.429, 2498 times serum dilution for B.1.525, 1801 times serum dilution for B.1.526, 28969 times serum dilution for B.1.1.7 (Alpha), 3462 times serum dilution for B.1.351 (Beta), 1899 times serum dilution for B.1.1.28 (Gamma), 15013 times serum dilution for B1.617.2 (Delta), 6276 times serum dilution for C.37 (Lambda), and 4250 times serum dilution for B.1.621 (Mu).

Especially the activity against Omicron mutant strain was high, with a serum dilution of 6616 times for B.1.1.529 (Omicron BA. 1), 2621 times for Omicron BA. 2, 1983 times for Omicron BA. 3, and 4984 times for Omicron BA.4/5. This indicates that the antibody of this protein has good activity against various SARS-CoV-2 mutant strains of viruses entering cells.

### Example 9: Antiserum titers of rabbit serum of novel coronavirus recombinant proteins HR121 and HR212 for the neutralization of SARS-CoV-2 virus

On a 48-well cell culture plate, 200 µl of HPAEpiC (5×105 pcs/ml) per well was added for 12 h, the supernatant was discarded, 100 µl of DMEM medium of serial dilutions of HR121 or HR212 anti-rabbit serum was added per well (triplicate wells per dilution), and then 100 µl SARS-CoV-2 virus at MOI of 1 was added. Simultaneously positive control wells without protein and negative control wells without virus were set. After incubating at 37 °C for 2 hours, the supernatant was discarded and the cells were washed with PBS for three times, then 200 µl of DMEM medium with HR121 or HR212 anti rabbit serum diluted in gradient was added. After incubating at 37°C in a CO₂ incubator for 48 h, the supernatant was taken and the RNA in the culture supernatant was extracted with the Roche virus RNA extraction kit. Quantitative PCR was used to detect the content of viral RNA in the supernatant, and the primers for quantitative PCR were:
SARS-CoV-2-NF: 5 '- GGGAACTTTCCTGCTAGAAT-3' (SEQ ID NO. 17);
SARS-CoV-2-NR: 5 '- CAGACATTTGCTCAAGCTG-3' (SEQ ID NO. 18);
SARS-CoV-2-N-probe: FAM-TTGCTGCTGCTTGACAGATT-TAMRA-3 '(SEQ ID NO. 19).

EC₅₀ values for HR121 or HR212 anti-rabbit serum are calculated based on the content of viral RNA.

The analysis and detection results are shown in Figure 7. The EC₅₀ of HR121 protein against rabbit serum neutralizing SARS-CoV-2 virus was 8515 times serum dilution. The EC₅₀ value of HR212 protein in anti-rabbit serum and anti-rabbit serum neutralizing SARS-CoV-2 virus is 1308 times serum dilution. This indicates that the anti-rabbit serum of these two proteins has good anti SARS-CoV-2 virus replication activity.

### Example 10: Purification of rabbit antibodies against recombinant fusion proteins HR121 and HR212 of novel coronavirus and determination of their anti SARS CoV-2 virus replication activity.

10.1 Purification of rabbit antibodies against the recombinant proteins HR121 and HR212 of novel coronavirus: The anti-rabbit serum of HR121 and HR212 was purified by protein A packing purification kit, and the anti-rabbit IgG polyclonal antibodies against HR121 and HR212 were obtained.

10.2 Determination of the inhibitory activity of HR121 and HR212 rabbit antibodies against SARS-CoV-2 virus replication: The detection method is the same as Example 9, except that the test sample "HR121 or HR212 anti rabbit serum diluted in gradient" was replaced with "HR121 or HR212 rabbit antibody diluted in gradient ".

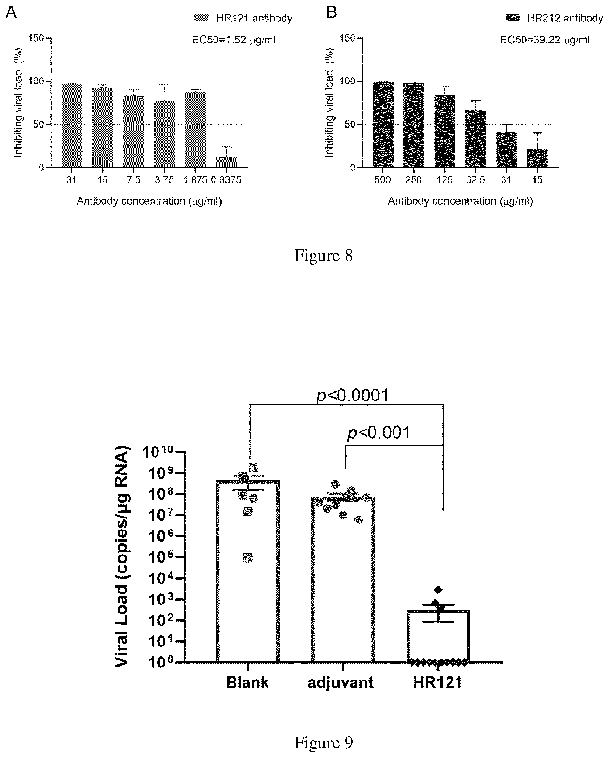

The results of the analysis are shown in Figure 8, and the EC₅₀ of the rabbit antibody against HR121 protein neutralizing the SARS-CoV-2 virus is 1.52 µg/mL. The EC₅₀ value of HR212 protein rabbit antibody neutralizing SARS-CoV-2 virus is 39.22 µg/mL. This indicates that the antibodies against these two proteins have good anti-SARS-CoV-2 virus replication activity.

### Example 11: Protective effect of recombinant protein HR121 of novel coronavirus as vaccine on golden hamsters infected with SARS CoV-2 virus

### (1) Protective effect of the novel coronavirus recombinant protein HR121 as a vaccine against golden hamsters infected with the original strain of SARS-CoV-2 virus

11.1. 8-week-old golden hamsters were immunized with Novel coronavirus recombinant protein HR121. Primary immunization: The recombinant protein HR121 prepared in Example 3 was diluted in PBS solution to achieve a protein concentration of 100 µg/mL. The same volume of Freund complete adjuvant was added and mixed with the protein solution to form a white latex. Each hamster was immunized with 0.3 ml via subcutaneous multipoint (protein content was 15 µg/ animal). Boost immunity: two booster immunizations were performed on day 14 and 28. The recombinant protein HR121 prepared in Example 3 was diluted in PBS solution to achieve a protein concentration of 100 µg/mL. The same volume of Freund's incomplete adjuvant was added and mixed with the protein solution to form a white latex. Each rabbit was immunized with 0.3 ml via subcutaneous multipoint (protein content was 15µg/ rabbit). At the same time, a negative control group without protein and a negative control group without adjuvant were set up.

11.2. Protective effect on the hamster: The hamster was attacked with SARS-CoV-2 virus at a dose of TCID50=104 at 42 days, and the hamster was dissected 3 days after the attack. Lung tissue was extracted with Trizol reagent. The content of viral genomic RNA(gRNA) in supernatant was detected by quantitative PCR. The primers of quantitative PCR were:
SARS-CoV-2-NF: 5 '- GGGAACTTTCCTGCTAGAAT-3' (SEQ ID NO. 17);
SARS-CoV-2-NR: 5 '- CAGACATTTGCTCAAGCTG-3' (SEQ ID NO. 18);
SARS-CoV-2-N-probe: FAM-TTGCTGCTGCTTGAGATT-TAMRA-3 '(SEQ ID NO. 19).

The protective effect of HR121 against hamsters infected by SARS-CoV-2 virus was calculated based on the content of viral RNA.

As shown in Figure 9, HR121 protein can protect golden hamsters against SARS-CoV-2 infection after immunizing golden hamsters, and was a candidate protein vaccine for preventing SARS-CoV-2 infection of novel coronavirus.

### (2) Protective effect of recombinant novel coronavirus protein HR121 as a vaccine against golden hamster infected with SARS-CoV-2 virus mutant Omicron BA.2

11.3 8-week-old golden hamsters were immunized with the novel coronavirus recombinant protein HR121. Primary immunization: The recombinant protein HR121 prepared in Example 3 was diluted with PBS solution to achieve a protein concentration of 100 µg/mL. The same volume of Freund's complete adjuvant or aluminum adjuvant was added and mixed with the protein solution to form a white latex, and each hamster was immunized with 0.3 ml (protein content 15µg/ animal) via multiple subcutaneous points (Freund's adjuvant group) or intramuscular injection (aluminum adjuvant group). Boost immunity: two booster immunizations were performed on day 14 and 28. The recombinant protein HR121 prepared in Example 3 was diluted with PBS solution to achieve a protein concentration of 100 µg/mL. The same volume of Freund's imcomplete adjuvant or aluminum adjuvant was added and mixed with the protein solution to form a white latex, and each rabbit was immunized with 0.3 ml (protein content 15µg/ animal) via multiple subcutaneous points (Freund's adjuvant group) or intramuscular injection (aluminum adjuvant group). Simultaneously, a negative control immune group without protein was set up.

11.4 Protective effect on hamster: The hamsters were attacked with SARS-CoV-2 virus at a dose of TCID50 = 104 at day 42, and the hamsters were dissected 3 days after the attack. Lung tissue was extracted with Trizol reagent. The viral RNA content in the supernatant was detected using quantitative PCR method. The primers for quantitative PCR of viral genomic RNA (gRNA) were:
SARS-CoV-2-NF: 5 '- GGGAACTTTCCTGCTAGAAT-3' (SEQ ID NO. 17);
SARS-CoV-2-NR: 5 '- CAGACATTTGCTCAAGCTG-3' (SEQ ID NO. 18);
SARS-CoV-2-N-probe: 5 '- FAM-TTGCTGCTTGACAGATT-TAMRA-3' (SEQ ID NO. 19).

Virus subgenome RNA (representing the replicating virus, sgRNA). The primers for quantitative PCR are:
SARS-CoV-2-EF: 5 '- CGATCTTTGTAGATTGTTCTC-3' (SEQ ID NO. 20);
SARS-CoV-2-ER: 5 '- ATATTGCAGCAGTACGCACACA -3' (SEQ ID NO. 21);
SARS-CoV-2-E-probe: 5'-FAM-CGAAGCGCAGTAAGGATGGCTAGTGT-TAMRA-3' (SEQ ID NO. 22).

The protective effect of HR121 against gopher infected by SARS-CoV-2 virus was calculated based on the content of gRNA and sgRNA.

The analysis and detection results are shown in Figure 14. After immunizing golden hamsters with HR121 protein, it can effectively protect golden hamsters from being infected by the SARS CoV-2 mutant Omicron BA.2, which is a good candidate protein vaccine for preventing the infection of the novel coronavirus SARS CoV-2 mutant.

### Example 12: Construction and purification of expression vectors for other HR121 and HR212 with partial deletion of amino acid sequences in HR1 and HR2 regions

Modification of the amino acid sequence of HR1 or HR2: 3 other HR121 proteins with missing amino acids: HR121-1, HR121-2, HR121-3 and 3 other HR212 proteins: HR212-1, HR212-2, HR212-3. The expression and purification method was the same as HR121 and HR212 in Example 2 .

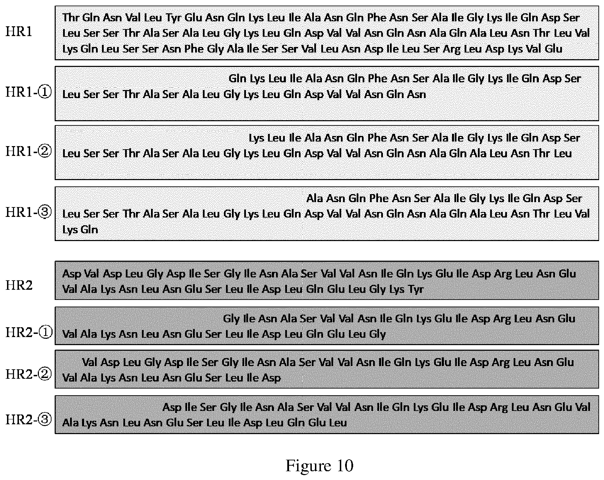

On the basis of HR1 amino acid sequence (SEQ ID NO.1) and HR2 amino acid sequence (SEQ ID NO.2), partial deletions were performed on the amino acid sequences of HR1 and HR2 regions, resulting in HR1- ①, HR1- ②, and HR1- ③, respectively. The amino acid sequences of HR1- ①, HR1- ②, and HR1- ③ are shown in SEQ ID NO.11-13, and the amino acid sequences of HR2- ①, HR2- ②, and HR2- ③ are shown in SEQ ID NO.14-16. The comparison of the missing amino acid sequences is shown in Figure 10.

Based on these HR1 and HR2 sequences, three other HR121 proteins and three HR212 proteins were constructed. They were named as HR121-1, HR121-2, HR121-3 and HR212-1, HR212-2, HR212-3 respectively. Among them, the amino acid sequence of HR121-1 is connected by HR1- ① Linker1-HR2- ① Linker2-HR1- ①; The amino acid sequence of HR121-2 is linked by HR1- (2) Linker1-HR2- (2) Linker2-HR1- (2); The amino acid sequence of HR121-3 is linked by HR1- ③ Linker1-HR2- ③ Linker2-HR1- ③. The amino acid sequence of HR212-1 is linked by HR2 ① - Linker2-HR1 ① - Linker1-HR2 ①; The amino acid sequence of HR212-2 is linked by HR2- ② Linker 1-HR1- ② Linker2-HR2- ②; The amino acid sequence of HR212-3 is linked by HR2- ③ Linker1-HR1- ③ Linker2-HR2- ③.

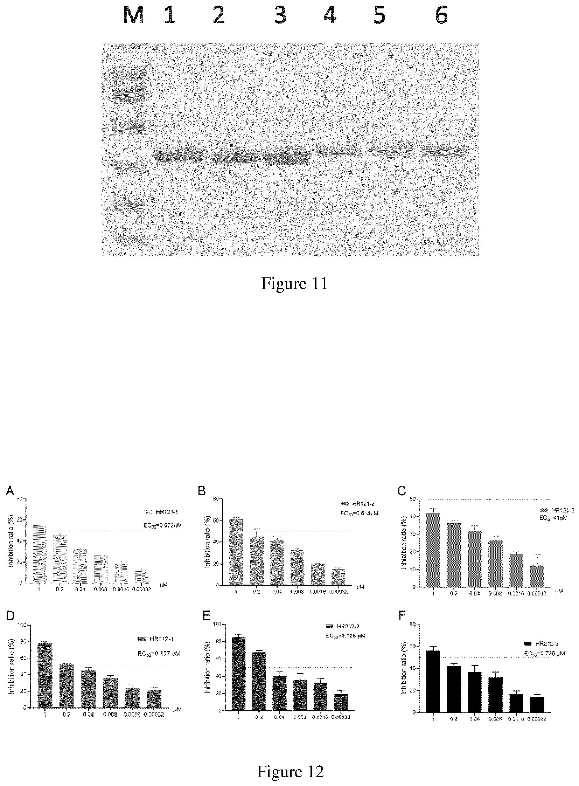

After their nucleotide coding sequences were connected into the pET-30a vector, the proteins were purified according to the method of Examples 1-3, and the SDS-PAGE electrophoresis diagram of the purified proteins was shown in FIG. 11. Among them, the size of HR121-1 protein is about 18kDa, HR121-2 protein is about 19kDa, and HR121-3 protein is about 19kDa. The size of HR212-1 protein is about 18kDa; The size of HR212-2 protein is about 19 kDa; The size of HR212-3 protein is about 19 kDa.

### Example 13: Other HR121 and HR212 with partial deletion of amino acid sequences in HR1 and HR2 regions also have antiviral activity.

According to the method of Example 5, the anti-novel coronavirus activities of the three HR121 and three HR212 were detected, and it was found that other HR121 and HR212 with partial deletion of amino acid sequence in HR1 and HR2 regions still had antiviral activity. The results are shown in FIG. 12, where the EC₅₀ value of HR121-1 anti-novel coronavirus activity is 0.672µM, the EC50 value of HR121-2 is 0.614µM, and the EC50 value of HR121-3 is <1µM. The EC₅₀ value of HR212-1's anti-novel coronavirus activity is 0.157 µ M. The EC₅₀ value of HR212-2 is 0.128 µ M. The EC₅₀ value of HR212-3 is 0.736 µ _{M}. The other three HR121 and three HR212 recombinant proteins with different amino acid sequences deleted in the HR1 and HR2 regions of novel coronavirus also have inhibitory activity against novel coronavirus.

The above results indicate that the other three HR121 and three HR212 recombinant proteins with different amino acid sequences deleted in the HR1 and HR2 regions of novel coronavirus can also mimic the conformation of the membrane fusion intermediate state of novel coronavirus, and have the activity of inhibiting the replication of novel coronavirus. Moreover, it can serve as an immunogen, inducing the production of strong neutralizing antibodies and exhibiting good immunogenicity.

The novel coronavirus recombinant protein HR121 or HR212 prepared in the invention is derived from the HR region of novel coronavirus S2 protein, and can simulate the intermediate conformation of novel coronavirus membrane fusion. These two proteins act on the membrane fusion phase of novel coronavirus, and have certain anti-novel coronavirus activity, which is expected to be used to prepare new broad-spectrum anti-novel coronavirus detection reagent materials and preventive or therapeutic drugs.

The recombinant protein HR121 or HR212 of novel coronavirus as immunogen can induce high affinity neutralizing antibodies against novel coronavirus in rabbits. After immunizing golden hamsters with recombinant protein HR121 of novel coronavirus, golden hamsters could be substantially protected from novel coronavirus infection. Its antibody activity and animal protection effect are comparable to the reported antibody activity induced by RBD vaccine derived from the novel coronavirus S1 protein (Nature.2020 Oct; 586(7830):572-577. doi: 10.1038/s41586-020-2599-8). Therefore, the novel coronavirus SARS-CoV-2 recombinant protein HR121 or HR212 prepared in the invention can have the application prospect of developing a new broad-spectrum novel coronavirus protein vaccine.

In conclusion, the recombinant protein HR121 or HR212 of novel coronavirus in some examples of the invention is expected to be used to prepare new raw materials for novel coronavirus detection reagents, drugs, vaccines and antibodies.

The sequences of the present invention were as follows:
SEQ ID NO. 1 The amino acid sequence of HR1 
TQNVLYENQKLIANQFNSAIGKIQDSLSSTASALGKLQDVVNQNAQALNTLVK
QLSSNFGAISSVLNDILSRLDKVE
SEQ ID NO. 2 The amino acid sequence of HR2
   DVDLGDISGINASVVNIQKEIDRLNEVAKNLNESLIDLQELGKY
SEQ ID NO. 3 Linker1 GGSGG
SEQ ID NO. 4 Linker2 SGGRGG
SEQ ID NO. 5 The amino acid sequence of HR121 
TQNVLYENQKLIANQFNSAIGKIQDSLSSTASALGKLQDVVNQNAQALNTLVK
QLSSNFGAISSVLNDILSRLDKVEGGSGGDVDLGDISGINASVVNIQKEIDRLNEVAKNL
NESLIDLQELGKYSGGRGGTQNVLYENQKLIANQFNSAIGKIQDSLSSTASALGKLQDVV
NQNAQALNTLVKQLSSNFGAISSVLNDILSRLDKVE
SEQ ID NO. 6 The amino acid sequence of HR212 
DVDLGDISGINASVVNIQKEIDRLNEVAKNLNESLIDLQELGKYSGGRGGTQNV
LYENQKLIANQFNSAIGKIQDSLSSTASALGKLQDVVNQNAQALNTLVKQLSSNFGAISS
VLNDILSRLDKVEGGSGGDVDLGDISGINASVVNIQKEIDRLNEVAKNLNESLIDLQELG
KY
SEQ ID NO. 7 Nucleotide sequence encoding HR121 
Acacagaatgttctctatgagaaccaaaaattgattgccaaccaatttaatagtgctattggcaaaattcaagactcactttcttcc
acagcaagtgcacttggaaaacttcaagatgtggtcaaccaaaatgcacaagctttaaacacgcttgttaaacaacttagctccaattttggtg
caatttcaagtgttttaaatgatatcctttcacgtcttgacaaagttgagggaggaagcggaggagatgttgatttaggtgacatctctggcatta
atgcttcagttgtaaacattcaaaaagaaattgaccgcctcaatgaggttgccaagaatttaaatgaatctctcatcgatctccaagaacttgga
aagtatagcggaggaagaggaggaacacagaatgttctctatgagaaccaaaaattgattgccaaccaatttaatagtgctattggcaaaatt
caagactcactttcttccacagcaagtgcacttggaaaacttcaagatgtggtcaaccaaaatgcacaagctttaaacacgcttgttaaacaac
ttagctccaattttggtgcaatttcaagtgttttaaatgatatcctttcacgtcttgacaaagttgagtaa
SEQ ID NO. 8 Nucleotide sequence encoding HR212 
Gatgttgatttaggtgacatctctggcattaatgcttcagttgtaaacattcaaaaagaaattgaccgcctcaatgaggttgccaa
gaatttaaatgaatctctcatcgatctccaagaacttggaaagtatagcggaggaagaggaggaacacagaatgttctctatgagaaccaaa
aattgattgccaaccaatttaatagtgctattggcaaaattcaagactcactttcttccacagcaagtgcacttggaaaacttcaagatgtggtca
accaaaatgcacaagctttaaacacgcttgttaaacaacttagctccaattttggtgcaatttcaagtgttttaaatgatatcctttcacgtcttgac
aaagttgagggaggaagcggaggagatgttgatttaggtgacatctctggcattaatgcttcagttgtaaacattcaaaaagaaattgaccgc
ctcaatgaggttgccaagaatttaaatgaatctctcatcgatctccaagaacttggaaagtattaa
SEQ ID NO. 9 Nucleotide sequence encoding HR1 
Acacagaatgttctctatgagaaccaaaaattgattgccaaccaatttaatagtgctattggcaaaattcaagactcactttcttcc
acagcaagtgcacttggaaaacttcaagatgtggtcaaccaaaatgcacaagctttaaacacgcttgttaaacaacttagctccaattttggtg
caatttcaagtgttttaaatgatatcctttcacgtcttgacaaagttgag
SEQ ID NO. 10 Nucleotide sequence encoding HR2 
Gatgttgatttaggtgacatctctggcattaatgcttcagttgtaaacattcaaaaagaaattgaccgcctcaatgaggttgccaa
gaatttaaatgaatctctcatcgatctccaagaacttggaaagtat
SEQ ID NO. 11 The amino acid sequence of HR1- ①
   QKLIANQFNSAIGKIQDSLSSTASALGKLQDVVNQN
SEQ ID NO. 12 The amino acid sequence of HR1- ②
   KLIANQFNSAIGKIQDSLSSTASALGKLQDVVNQNAQALNTL
SEQ ID NO. 13 The amino acid sequence of HR1- ③
   ANQFNSAIGKIQDSLSSTASALGKLQDVVNQNAQALNTLV
SEQ ID NO. 14 The amino acid sequence of HR2- ①
   GINASVVNIQKEIDRLNEVAKNLNESLIDLQELG
SEQ ID NO. 15 The amino acid sequence of HR2- ②
   VDLGDISGINASVVNIQKEIDRLNEVAKNLNESLID
SEQ ID NO. 16 The amino acid sequence of HR2- ③
   DISGINASVVNIQKEIDRLNEVAKNLNESLIDLQEL
SEQ ID NO. 17 SARS-CoV-2-NF GGGGAACTTCTCCTGCTAGAAT
SEQ ID NO. 18 SARS-CoV-2-NR CAGACATTTTGCTCTCAAGCTG
SEQ ID NO. 19 SARS-CoV-2-N-probe TTGCTGCTGCTTGACAGATT
SEQ ID NO. 20 SARS-CoV-2-EF CGATCTCTTGTAGATCTGTTCTC
SEQ ID NO. 21 SARS-CoV-2-ER ATATTGCAGCAGTACGCACACA
SEQ ID NO. 22 SARS-CoV-2-E-probe
   CGAAGCGCAGTAAGGATGGCTAGTGT
SEQ ID NO. 23 mRNA sequence encoding HR121 
AUGACACAGAAUGUUCUCUAUGAGAACCAAAAAUUGAUUGCCAACCAAUUU
AAUAGUGCUAUUGGCAAAAUUCAAGACUCACUUUCUUCCACAGCAAGUGCACUU
GGAAAACUUCAAGAUGUGGUCAACCAAAAUGCACAAGCUUUAAACACGCUUGUU
AAACAACUUAGCUCCAAUUUUGGUGCAAUUUCAAGUGUUUUAAAUGAUAUCCUU
UCACGUCUUGACAAAGUUGAGGGAGGAAGCGGAGGAGAUGUUGAUUUAGGUGAC
AUCUCUGGCAUUAAUGCUUCAGUUGUAAACAUUCAAAAAGAAAUUGACCGCCUC
AAUGAGGUUGCCAAGAAUUUAAAUGAAUCUCUCAUCGAUCUCCAAGAACUUGGA
AAGUAUAGCGGAGGAAGAGGAGGAACACAGAAUGUUCUCUAUGAGAACCAAAAA
UUGAUUGCCAACCAAUUUAAUAGUGCUAUUGGCAAAAUUCAAGACUCACUUUCU
UCCACAGCAAGUGCACUUGGAAAACUUCAAGAUGUGGUCAACCAAAAUGCACAA
GCUUUAAACACGCUUGUUAAACAACUUAGCUCCAAUUUUGGUGCAAUUUCAAGU
GUUUUAAAUGAUAUCCUUUCACGUCUUGACAAAGUUGAGUAA
SEQ ID NO. 24 mRNA sequence encoding HR121-1 
AUGCAAAAAUUGAUUGCCAACCAAUUUAAUAGUGCUAUUGGCAAAAUUC
AAGACUCACUUUCUUCCACAGCAAGUGCACUUGGAAAACUUCAAGAUGUGGUCA
ACCAAAAUGGAGGAAGCGGAGGAGGCAUUAAUGCUUCAGUUGUAAACAUUCAAA
AAGAAAUUGACCGCCUCAAUGAGGUUGCCAAGAAUUUAAAUGAAUCUCUCAUCG
AUCUCCAAGAACUUGGAAGCGGAGGAAGAGGAGGACAAAAAUUGAUUGCCAACC
AAUUUAAUAGUGCUAUUGGCAAAAUUCAAGACUCACUUUCUUCCACAGCAAGUG
CACUUGGAAAACUUCAAGAUGUGGUCAACCAAAAUUAA
SEQ ID NO. 25 mRNA sequence encoding HR121-2 
AUGAAAUUGAUUGCCAACCAAUUUAAUAGUGCUAUUGGCAAAAUUCAAG
ACUCACUUUCUUCCACAGCAAGUGCACUUGGAAAACUUCAAGAUGUGGUCAACCA
AAAUGCACAAGCUUUAAACACGCUUGGAGGAAGCGGAGGAGUUGAUUUAGGUGA
CAUCUCUGGCAUUAAUGCUUCAGUUGUAAACAUUCAAAAAGAAAUUGACCGCCU
CAAUGAGGUUGCCAAGAAUUUAAAUGAAUCUCUCAUCGAUAGCGGAGGAAGAGG
AGGAAAAUUGAUUGCCAACCAAUUUAAUAGUGCUAUUGGCAAAAUUCAAGACUC
ACUUUCUUCCACAGCAAGUGCACUUGGAAAACUUCAAGAUGUGGUCAACCAAAA
UGCACAAGCUUUAAACACGCUUUAA
SEQ ID NO. 26 mRNA sequence encoding HR121-3 
AUGGCCAACCAAUUUAAUAGUGCUAUUGGCAAAAUUCAAGACUCACUUU
CUUCCACAGCAAGUGCACUUGGAAAACUUCAAGAUGUGGUCAACCAAAAUGCACA
AGCUUUAAACACGCUUGUUAAACAAGGAGGAAGCGGAGGAGACAUCUCUGGCAU
UAAUGCUUCAGUUGUAAACAUUCAAAAAGAAAUUGACCGCCUCAAUGAGGUUGC
CAAGAAUUUAAAUGAAUCUCUCAUCGAUCUCCAAGAACUUAGCGGAGGAAGAGG
AGGAGCCAACCAAUUUAAUAGUGCUAUUGGCAAAAUUCAAGACUCACUUUCUUC
CACAGCAAGUGCACUUGGAAAACUUCAAGAUGUGGUCAACCAAAAUGCACAAGC
UUUAAACACGCUUGUUAAACAAUAA
SEQ ID NO. 27 mRNA sequence encoding HR212 
AUGGAUGUUGAUUUAGGUGACAUCUCUGGCAUUAAUGCUUCAGUUGUAAAC
AUUCAAAAAGAAAUUGACCGCCUCAAUGAGGUUGCCAAGAAUUUAAAUGAAUCU
CUCAUCGAUCUCCAAGAACUUGGAAAGUAUAGCGGAGGAAGAGGAGGAACACAG
AAUGUUCUCUAUGAGAACCAAAAAUUGAUUGCCAACCAAUUUAAUAGUGCUAUU
GGCAAAAUUCAAGACUCACUUUCUUCCACAGCAAGUGCACUUGGAAAACUUCAAG
AUGUGGUCAACCAAAAUGCACAAGCUUUAAACACGCUUGUUAAACAACUUAGCU
CCAAUUUUGGUGCAAUUUCAAGUGUUUUAAAUGAUAUCCUUUCACGUCUUGACA
AAGUUGAGGGAGGAAGCGGAGGAGAUGUUGAUUUAGGUGACAUCUCUGGCAUUA
AUGCUUCAGUUGUAAACAUUCAAAAAGAAAUUGACCGCCUCAAUGAGGUUGCCA
AGAAUUUAAAUGAAUCUCUCAUCGAUCUCCAAGAACUUGGAAAGUAUUAA
SEQ ID NO. 28 mRNA sequence encoding HR212-1 
AUGGGCAUUAAUGCUUCAGUUGUAAACAUUCAAAAAGAAAUUGACCGCC
UCAAUGAGGUUGCCAAGAAUUUAAAUGAAUCUCUCAUCGAUCUCCAAGAACUUG
GAAGCGGAGGAAGAGGAGGACAAAAAUUGAUUGCCAACCAAUUUAAUAGUGCUA
UUGGCAAAAUUCAAGACUCACUUUCUUCCACAGCAAGUGCACUUGGAAAACUUCA
AGAUGUGGUCAACCAAAAUGGAGGAAGCGGAGGAGGCAUUAAUGCUUCAGUUGU
AAACAUUCAAAAAGAAAUUGACCGCCUCAAUGAGGUUGCCAAGAAUUUAAAUGA
AUCUCUCAUCGAUCUCCAAGAACUUGGAUAA
SEQ ID NO. 29 mRNA sequence encoding HR212-2 
AUGGUUGAUUUAGGUGACAUCUCUGGCAUUAAUGCUUCAGUUGUAAACA
UUCAAAAAGAAAUUGACCGCCUCAAUGAGGUUGCCAAGAAUUUAAAUGAAUCUC
UCAUCGAUAGCGGAGGAAGAGGAGGAAAAUUGAUUGCCAACCAAUUUAAUAGUG
CUAUUGGCAAAAUUCAAGACUCACUUUCUUCCACAGCAAGUGCACUUGGAAAACU
UCAAGAUGUGGUCAACCAAAAUGCACAAGCUUUAAACACGCUUGGAGGAAGCGG
AGGAGUUGAUUUAGGUGACAUCUCUGGCAUUAAUGCUUCAGUUGUAAACAUUCA
AAAAGAAAUUGACCGCCUCAAUGAGGUUGCCAAGAAUUUAAAUGAAUCUCUCAU
CGAUUAA
SEQ ID NO. 30 mRNA sequence encoding HR212-3

AUGGACAUCUCUGGCAUUAAUGCUUCAGUUGUAAACAUUCAAAAAGAAA
UUGACCGCCUCAAUGAGGUUGCCAAGAAUUUAAAUGAAUCUCUCAUCGAUCUCCA
AGAACUUAGCGGAGGAAGAGGAGGAGCCAACCAAUUUAAUAGUGCUAUUGGCAA
AAUUCAAGACUCACUUUCUUCCACAGCAAGUGCACUUGGAAAACUUCAAGAUGU
GGUCAACCAAAAUGCACAAGCUUUAAACACGCUUGUUAAACAAGGAGGAAGCGG
AGGAGACAUCUCUGGCAUUAAUGCUUCAGUUGUAAACAUUCAAAAAGAAAUUGA
CCGCCUCAAUGAGGUUGCCAAGAAUUUAAAUGAAUCUCUCAUCGAUCUCCAAGAA
CUUUAA

For professionals in this field to easily understand, the above is only a preferred embodiment of the present invention patent and is not intended to limit the present invention. Any modification, equivalent replacement and improvement made within the spirit and principle of the invention fall within the protection scope of the invention. For example, the deletion or modification of the amino acid of the HR1 or HR2 peptide segment in the above embodiment, the change of the host cell into yeast cell, Escherichia coli or mammalian cell, etc., so that the corresponding skeleton carrier is selected according to the host cell, the expression of novel coronavirus recombinant protein HR121 or HR212 similar to the above experimental examples, and the use of the novel coronavirus recombinant protein HR121 or HR212 as a vaccine or the preparation of its antibodies fall within the protection scope of the invention.

## Claims

1. A recombinant fusion protein from the HR region of novel coronavirus S2 protein, wherein the novel coronavirus recombinant fusion protein HR121 or HR212 is composed of a truncated protein HR1 derived from the HR1 region of the novel coronavirus S2 protein, a truncated protein HR2 derived from the HR2 region, and a linking peptide conjugating them;
the protein structure of HR121 is HR1-Linker1-HR2-linker2-HR1;
the protein structure of HR212 is HR2-Linker2-HR1-Linker1-HR2;
wherein the HR1 amino acid sequence is as shown in SEQ ID NO.1 or comprises a sequence that one or more amino acids are substituted, deleted, or inserted in the amino acid sequence shown in SEQ ID NO.1;
the HR2 amino acid sequence is as shown in SEQ ID NO.2 or comprises a sequence that one or more amino acids are substituted, deleted, or inserted in the amino acid sequence shown in SEQ ID NO.2;
the amino acid sequences of Linker1 and Linker2 are shown in SEQ ID NOs.3-4, respectively.

2. The recombinant fusion protein from the HR region of novel coronavirus S2 protein of claim 1, wherein the amino acid sequence of the novel coronavirus recombinant fusion protein HR121 is shown in SEQ ID NO.5.

3. The recombinant fusion protein from the HR region of novel coronavirus S2 protein of claim 1, wherein the amino acid sequence of the novel coronavirus recombinant fusion protein HR212 is shown in SEQ ID NO.6.

4. The recombinant fusion protein from the HR region of novel coronavirus S2 protein of claim 2, wherein the nucleotide sequence encoding HR121 is shown in SEQ ID NO.7.

5. The recombinant fusion protein from the HR region of novel coronavirus S2 protein of claim 3, wherein the nucleotide sequence encoding HR212 is shown in SEQ ID NO.8.

6. A vector expressing the recombinant fusion protein from the HR region of novel coronavirus S2 protein, wherein the vector is a HR121 skeleton vector, a HR212 skeleton vector, and the HR121 skeleton vector and HR212 skeleton vector comprise the nucleotide sequences of claims 4 and 5, respectively.

7. Use of the recombinant fusion protein from the HR region of novel coronavirus S2 protein of any one of claims 1-5 in the manufacture of an antigen detection reagent and a medicament fo prevention or treatment of novel coronavirus.

8. Use of the recombinant fusion protein from the HR region of novel coronavirus S2 protein of any one of claims 1-5 as an immunogen in the manufacture of a vaccine and an antibody for novel coronavirus.
